# EUROPEAN PATENT APPLICATION

(11) **EP 1 605 064 A1**
(43) Date of publication of application: **14.12.2005**
(21) Application number: 04076669.3
(22) Date of filing: 07.06.2004
(51) Int. Cl.: C12Q 1/70, C12Q 1/68, C12N 15/48

(54) **HIV Variant showing a novel resistance mechanism against protease inhibitors, diagnostic assays for detecting the variant, and methods for identifying drugs effective against the resistant virus**

(71) Applicant: UMC Utrecht Holding B.V., 3584 CM Utrecht (NL)
(72) Inventor: Boucher, Charles Achim Bernard, 3512 EH Utrecht (NL); Schipper, Pauline Johanna, 3448 GM Woerden (NL); van Maarseveen, Noortje Marianne, 3564 KZ Utrecht (NL); Nijhuis, Monique Johanna Gezina, 6913 KC Aerdt (NL)
(74) Representative: Van Someren, Petronella F. H. M.

(57) **Abstract**

The present invention relates to a novel Human Immunodeficiency Virus 1 (HIV-1) variant that is resistant against the high genetic barrier protease inhibitor RO033-4649 and shows mutations not in the protease itself but in its cleavage sites thus establishing a novel resistance mechanism. The invention further relates to diagnostic assays for determining the presence in a sample of the HIV variant.

SEQUENCE LISTING

<110> UMC Utrecht Holding B.V.
      Schipper, Pauline Johanna
      van Maarseveen, Noortje Marianne
      Nijhuis, Monique Johanna Gezina
      Boucher, Charles Achim Bernard

<120> HIV variant showing a novel resistance mechanism against protease
       inhibitors, diagnostic assays for detecting the variant, and
      methods for identifying drugs effective against the resistance
      variant

<130> L/2BB16/10

<140> EP 2004076669.3
<141> 2004-06-09

<160> 60

<170> PatentIn version 3.2

<210> 1
<211> 78
<212> DNA
<213> Human immunodeficiency virus type 1

<400> 1
gagagacagg ctaatttttt agggaagatc tggccttcct acaagggaag gccagggaat      60

tttcttcaga gcagacca                                                    78


<210> 2
<211> 78
<212> DNA
<213> Human immunodeficiency virus type 1

<400> 2
gagagacagg ctaatttttt aggggagacc tggccttcct acaagggaag gccagggaat      60

tttcttcaga gcagacca                                                    78


<210> 3
<211> 78
<212> DNA
<213> Human immunodeficiency virus type 1

<400> 3
gagagacagg ctaatttttt agggaaggtc tggccttcct acaagggaag gccagggaat      60

tttcttcaga gcagacca                                                    78


<210> 4
<211> 78

<212>  DNA
<213>  Human immunodeficiency virus type 1

<400>  4
gagagacagg ctaatttttt agggaagacc tggccttcct acaagggaag gccagggaat      60

tttcttcaga gcagacca                                                    78


<210>  5
<211>  78
<212>  DNA
<213>  Human immunodeficiency virus type 1

<400>  5
gagagacagg ctaatttttt aggggaggtc tggccttcct acaagggaag gccagggaat      60

tttcttcaga gcagacca                                                    78


<210>  6
<211>  63
<212>  DNA
<213>  Human immunodeficiency virus type 1

<400>  6
ttttttaggg aagatctggc cttcctacaa gggaaggcca gggaattttc ttcagagcag      60

acc                                                                    63


<210>  7
<211>  63
<212>  DNA
<213>  Human immunodeficiency virus type 1

<400>  7
ttttttaggg gagacctggc cttcctacaa gggaaggcca gggaattttc ttcagagcag      60

acc                                                                    63


<210>  8
<211>  63
<212>  DNA
<213>  Human immunodeficiency virus type 1

<400>  8
ttttttaggg aaggtctggc cttcctacaa gggaaggcca gggaattttc ttcagagcag      60

acc                                                                    63


<210>  9
<211>  63
<212>  DNA
<213>  Human immunodeficiency virus type 1

<400>  9
ttttttaggg aagacctggc cttcctacaa gggaaggcca gggaattttc ttcagagcag      60

acc                                                                   63


<210>  10
<211>  63
<212>  DNA
<213>  Human immunodeficiency virus type 1

<400>  10
ttttttaggg gaggtctggc cttcctacaa gggaaggcca gggaattttc ttcagagcag      60

acc                                                                   63


<210>  11
<211>  22
<212>  DNA
<213>  Artificial

<220>
<223>  primer 5'-clea-2

<400>  11
aaatgatgac agcatgtcag gg                                              22


<210>  12
<211>  30
<212>  DNA
<213>  Artificial

<220>
<223>  primer 3'-prot-2

<400>  12
aatgctttta ttttttcttc tgtcaatggc                                       30


<210>  13
<211>  23
<212>  DNA
<213>  Artificial

<220>
<223>  primer 5'-clea-1

<400>  13
gatgacagaa accttgttgg tcc                                             23


<210>  14
<211>  32
<212>  DNA

<213>  Artificial

<220>
<223>  primer 3'-prot-1

<400>  14
gcaaatactg gagtattgta tggattttca gg                                    32


<210>  15
<211>  25
<212>  DNA
<213>  Artificial

<220>
<223>  primer 5'-p2

<400>  15
aagcaatgag ccaggtaacc aattc                                            25


<210>  16
<211>  20
<212>  DNA
<213>  Artificial

<220>
<223>  primer 5'-MA-1

<400>  16
tagtatgggc aagcagggag                                                  20


<210>  17
<211>  24
<212>  DNA
<213>  Artificial

<220>
<223>  primer 3'-MA-3

<400>  17
catccatcct atttgttcct gaag                                             24


<210>  18
<211>  20
<212>  DNA
<213>  Artificial

<220>
<223>  primer 5'-NEF-1

<400>  18
gcagtagctg aggggacaga                                                  20

<210>  19
<211>  19
<212>  DNA
<213>  Artificial

<220>
<223>  primer 3'-NEF-1

<400>  19
ccctggtgtg tagttctgc                                                    19


<210>  20
<211>  29
<212>  DNA
<213>  Artificial

<220>
<223>  primer 5'RT-INT3

<400>  20
tattatgacc catcaaaaga cttaatagc                                         29


<210>  21
<211>  27
<212>  DNA
<213>  Artificial

<220>
<223>  primer 3'RT-INT3

<400>  21
aatgtgtaca atctagttgc catattc                                           27


<210>  22
<211>  23
<212>  DNA
<213>  Artificial

<220>
<223>  primer HXB2 startfor

<400>  22
gctacctagg gaacccactg ctt                                               23


<210>  23
<211>  27
<212>  DNA
<213>  Artificial

<220>
<223>  primer HBX2 gagrev

<400>  23

tgtgtttagc atggtgttta aatcttg 27

<210> 24
<211> 21
<212> DNA
<213> Artificial

<220>
<223> primer cleams-1

<400> 24
gaaggagcca ccccacaaga t 21

<210> 25
<211> 23
<212> DNA
<213> Artificial

<220>
<223> primer cleamsrev-1

<400> 25
ccttatggcc gggtcctcct act 23

<210> 26
<211> 20
<212> DNA
<213> Artificial

<220>
<223> primer ENV-5

<400> 26
ctgacggtac aggccagaca 20

<210> 27
<211> 20
<212> DNA
<213> Artificial

<220>
<223> primer NEF-3

<400> 27
tttgaccact tgccacccat 20

<210> 28
<211> 17
<212> DNA
<213> Artificial

<220>

<223>  primer 5-clea-4

<400>  28
ataatgatgc agagagg                                                    17


<210>  29
<211>  25
<212>  DNA
<213>  Artificial

<220>
<223>  primer 3'CS-2

<400>  29
tctcttctgg tggggctgtt ggctc                                           25


<210>  30
<211>  18
<212>  DNA
<213>  Artificial

<220>
<223>  primer PR5

<400>  30
agccaacagc cccaccag                                                   18


<210>  31
<211>  18
<212>  DNA
<213>  Artificial

<220>
<223>  primer PR2

<400>  31
cttttgggcc atccattc                                                   18


<210>  32
<211>  28
<212>  DNA
<213>  Artificial

<220>
<223>  primer 5'-MA-2

<400>  32
tataatacag tagcaaccct ctattgtg                                        28


<210>  33
<211>  23
<212>  DNA

<213>  Artificial

<220>
<223>  primer 3'-MA-2

<400>  33
tctgcagctt cctcattgat ggt                                                23


<210>  34
<211>  20
<212>  DNA
<213>  Artificial

<220>
<223>  primer 5'-NEF-2

<400>  34
atgggtggca agtggtcaaa                                                    20


<210>  35
<211>  20
<212>  DNA
<213>  Artificial

<220>
<223>  primer 3'-NEF-2

<400>  35
   tccagtcccc cctttctttt                                                   20


<210>  36
<211>  20
<212>  DNA
<213>  Artificial

<220>
<223>  primer 5'RT-INT2

<400>  36
agccacctgg attcctgagt                                                    20


<210>  37
<211>  20
<212>  DNA
<213>  Artificial

<220>
<223>  primer 3'RT-INT2

<400>  37
atcacagctg gctactattt                                                    20

<210>  38
<211>  23
<212>  DNA
<213>  Artificial

<220>
<223>  primer S667

<400>  38
cttaagcctc aaataagctt gaa                                              23


<210>  39
<211>  20
<212>  DNA
<213>  Artificial

<220>
<223>  primer 5'LTR-3

<400>  39
cagccttctg atgtttctaa                                                  20


<210>  40
<211>  24
<212>  DNA
<213>  Artificial

<220>
<223>  primer SK145

<400>  40
agtgggggga catcaagcag ccat                                             24


<210>  41
<211>  24
<212>  DNA
<213>  Artificial

<220>
<223>  primer cleamsrev-2

<400>  41
ttcttctagt gtagccgctg gtcc                                             24


<210>  42
<211>  20
<212>  DNA
<213>  Artificial

<220>
<223>  primer ENV-6

<400>  42

acaattattg tctggtatag                                                20

<210>  43
<211>  20
<212>  DNA
<213>  Artificial

<220>
<223>  primer ENV-7

<400>  43
cctttccaag ccctgtctta                                                20

<210>  44
<211>  22
<212>  DNA
<213>  Artificial

<220>
<223>  primer ENV-8

<400>  44
tatccctgcc taactctatt ca                                             22

<210>  45
<211>  27
<212>  DNA
<213>  Artificial

<220>
<223>  primer ENV-9

<400>  45
gtttaagaat agtttttgct gtacttt                                        27

<210>  46
<211>  23
<212>  DNA
<213>  Artificial

<220>
<223>  primer INT-Psh AI

<400>  46
gcatggacaa gtagactgta gtc                                            23

<210>  47
<211>  20
<212>  DNA
<213>  Artificial

<220>

<223>  primer INT-Nco I

<400>  47
tgttgcccta agccatggag                                                    20


<210>  48
<211>  24
<212>  DNA
<213>  Artificial

<220>
<223>  primer INT-Mlu NI

<400>  48
tgtattgttt ttaccggcca tctt                                               24


<210>  49
<211>  34
<212>  DNA
<213>  Artificial

<220>
<223>  primer p24 WT

<400>  49
gcaggaacta ctagtaccct tcaggaacaa atag                                    34


<210>  50
<211>  27
<212>  DNA
<213>  Artificial

<220>
<223>  primer p2 Bst EII Mut

<400>  50
tagctgaatt ggttacctgg ctcattg                                            27


<210>  51
<211>  26
<212>  PRT
<213>  Human immunodeficiency virus type 1

<400>  51

Glu Arg Gln Ala Asn Phe Leu Gly Lys Ile Trp Pro Ser Tyr Lys Gly
1               5                   10                  15


Arg Pro Gly Asn Phe Leu Gln Ser Arg Pro
            20                  25

<210>  52
<211>  26
<212>  PRT
<213>  Human immunodeficiency virus type 1

<400>  52

Glu Arg Gln Ala Asn Phe Leu Gly Glu Thr Trp Pro Ser Tyr Lys Gly
1               5                   10                  15

Arg Pro Gly Asn Phe Leu Gln Ser Arg Pro
            20                  25

<210>  53
<211>  26
<212>  PRT
<213>  Human immunodeficiency virus type 1

<400>  53

Glu Arg Gln Ala Asn Phe Leu Gly Lys Val Trp Pro Ser Tyr Lys Gly
1               5                   10                  15

Arg Pro Gly Asn Phe Leu Gln Ser Arg Pro
            20                  25

<210>  54
<211>  26
<212>  PRT
<213>  Human immunodeficiency virus type 1

<400>  54

Glu Arg Gln Ala Asn Phe Leu Gly Lys Thr Trp Pro Ser Tyr Lys Gly
1               5                   10                  15

Arg Pro Gly Asn Phe Leu Gln Ser Arg Pro
            20                  25

<210>  55
<211>  26
<212>  PRT
<213>  Human immunodeficiency virus type 1

<400>  55

Glu Arg Gln Ala Asn Phe Leu Gly Glu Val Trp Pro Ser Tyr Lys Gly
1               5                   10                  15

Arg Pro Gly Asn Phe Leu Gln Ser Arg Pro

            20                  25

<210> 56
<211> 21
<212> PRT
<213> Human immunodeficiency virus type 1

<400> 56

Phe Phe Arg Glu Asp Leu Ala Phe Leu Gln Lys Ala Arg Glu Phe Ser
1               5                   10                  15

Ser Glu Gln Thr Arg
            20

<210> 57
<211> 21
<212> PRT
<213> Human immunodeficiency virus type 1

<400> 57

Phe Phe Arg Gly Asp Leu Ala Phe Leu Gln Lys Ala Arg Glu Phe Ser
1               5                   10                  15

Ser Glu Gln Thr Arg
            20

<210> 58
<211> 21
<212> PRT
<213> Human immunodeficiency virus type 1

<400> 58

Phe Phe Arg Glu Gly Leu Ala Phe Leu Gln Lys Ala Arg Glu Phe Ser
1               5                   10                  15

Ser Glu Gln Thr Arg
            20

<210> 59
<211> 21
<212> PRT
<213> Human immunodeficiency virus type 1

<400> 59

Phe Phe Arg Glu Asp Leu Ala Phe Leu Gln Lys Ala Arg Glu Phe Ser
1               5                   10                  15

Ser Glu Gln Thr Arg
            20

<210>  60
<211>  21
<212>  PRT
<213>  Human immunodeficiency virus type 1

<400>  60

Phe Phe Arg Gly Gly Leu Ala Phe Leu Gln Lys Ala Arg Glu Phe Ser
1               5                   10                  15

Ser Glu Gln Thr Arg
            20

## Description

The present invention relates to new Human Immunodeficiency Virus 1 (HIV-1) variants in isolated form having a novel type of drug resistance and to the use of the genotypic characterization of these new resistant HIV-1 variants for the design of diagnostic assays and to diagnostic assays thus designed. The invention also relates to the use of the mutational profile of the variants for drug development, to the drugs thus developed and to their use in therapy. In addition, the invention relates to methods for evaluating the effectiveness of antiviral therapy in HIV-infected subjects. The invention also relates to a method for inducing resistance in HIV.

Human immunodeficiency virus 1 (HIV-1) is the causative agent of acquired immunodeficiency syndrome (AIDS). Initially the disease caused high morbidity and mortality rates. Since the mid-1990s treatment of HIV disease has changed dramatically with the advent of combination anti-retroviral therapy, the introduction of protease inhibitors (PIs), and the availability of viral load testing. With the use of these new tools, clinical outcomes have improved greatly.

Combination anti-retroviral therapy that includes protease inhibitors represents a significant advance in the therapy of HIV infection. HIV protease inhibitors may be combined with HIV inhibitors of other classes, e. g. fusion inhibitors, nucleoside reverse transcriptase inhibitors or non-nucleoside reverse transcriptase inhibitors, or used with other HIV protease inhibitors, often also with nucleoside reverse transcriptase inhibitors and/or non-nucleoside reverse transcriptase inhibitors, in so-called "cocktails".

However, although the use of combinations of anti-retroviral drugs has proven remarkably effective in controlling the progression of HIV disease and prolonging survival, these benefits can be compromised by the development of drug resistance. Resistance is the consequence of mutations that emerge in the viral proteins targeted by anti-retroviral agents.

Initially during anti-retroviral therapy, viral variants harbouring just 1-2 mutations in the target gene are being selected. Subsequently, during continuous therapy additional mutations in the target gene are observed. This clearly demonstrates that the viral population in the patient can accommodate only viral variants with a limited set of mutations.

HIV drug resistance mechanisms described so far involve mutations in this viral target gene of the drug. The concept of high genetic barrier (HGB) drugs is based on the insight that the variation in the viral quasispecies is limited to one or two mutations. During HGB therapy the virus requires far more target gene mutations than are normally present in the quasispecies making resistance escape difficult. Nevertheless, one can hypothesize that HIV, like other microorganisms could exploit alternative resistance mechanisms.

It is therefore a first object of the present invention to provide HIV-1 variants that show alternative drug resistance mechanisms against at least High Genetic Barrier drugs.

It is a further object of the invention to develop diagnostic assays and methods for monitoring the effectiveness of anti-retroviral therapy that are based on the information obtained from the study of such alternative drug resistance mechanisms.

In addition, the invention has for its object to use the information obtained from the study of such alternative drug resistance mechanisms in the development of new drugs.

To investigate the potential of alternative drug resistance mechanisms the present inventors have performed in vitro selection experiments using a HGB Protease Inhibitor (PI). To identify novel drug resistance pathways, multiple in vitro selection experiments were performed. Therefore, HIV-1 HXB2 was cultured in SupT1 cells in the presence of increasing levels of the HGB PI RO033-4649 (obtainable from Roche, Switzerland). RO033-4649 is a substrate based inhibitor that is active against drug resistant variants with 1-2 target gene mutations and against wild type HIV. Genotypic and phenotypic drug susceptibility analysis was performed.

In the presence of increasing drug concentrations the inventors were able to select a viral population displaying 6-8 fold resistance to the HGB drug RO033-4649 and to other PI. Interestingly, the viral population harboured no mutations in the viral protease. Sequence analysis of the viral gag gene demonstrated several nucleotide mixtures (1126, (matrix), 1460, (capsid) and 2180 (p6gag)). Three nucleotide changes were observed at position 907, 2095 and 2099. Interestingly, repeat experiments also demonstrated nucleotide changes in the same regions, at nucleotides 2098 or 2099. These changes are located in the ribosomal frameshift site, responsible for the production of the pol gene products. In addition, the same region codes for the transframe protein (TFP) and the p7/p1, p7/TFP and TFP/p6pol protease cleavage sites. It was highly surprising that these mutations, that were found at the location of the cleavage sites (CS), were not accompanied by mutations in the protease itself and were still effective.

*In vitro* selection experiments using the HGB protease inhibitor Ro-033-4649 resulted in the selection of a viral population displaying 6-8 fold PI resistance without mutations in the viral protease. This clearly demonstrates that a novel alternative drug resistance mechanism was identified. Interestingly, reproducible nucleotide changes in the region coding for the ribosomal frameshift site, the TFP and protease cleavage sites were observed that may be responsible for the reduced drug susceptibility.

The invention thus relates to Human Immunodeficiency Virus-1 (HIV-1) variants in isolated form that show a novel resistance mechanism against protease inhibitors and having at least one or more of the following mutations at the DNA level as compared to HIV-1 HXB2: A2095G, A2098G and T2099C.

At the amino acid level the HIV variant has at least one or more of the following mutations at the amino acid level as compared to HIV-1 HXB2: K436E in p1, I437T or I437V in p1. The same nucleotide changes also confer amino acid changes in the other translational frame: E4G and D5G (TransFrameProtein).

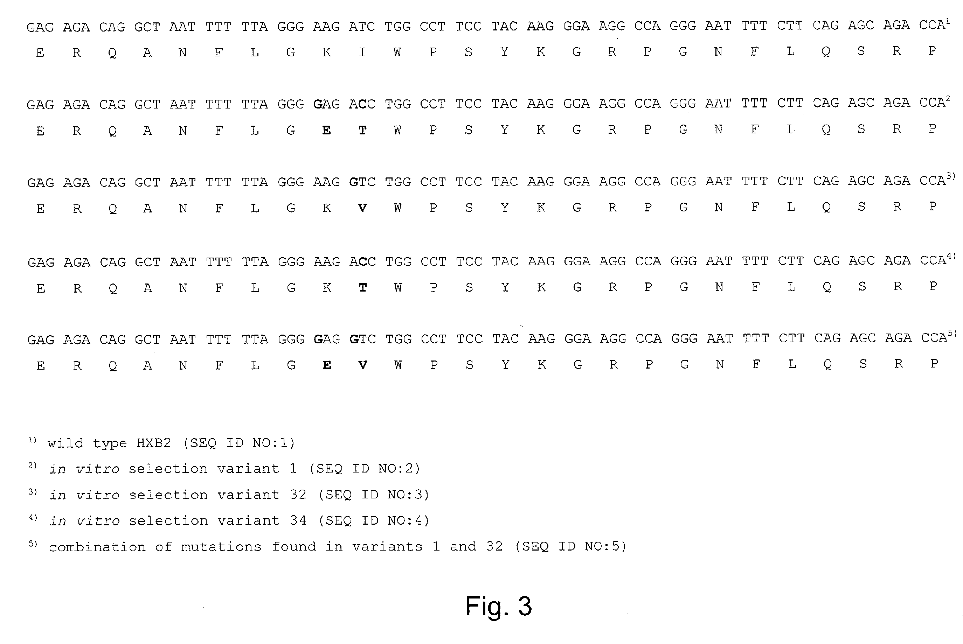

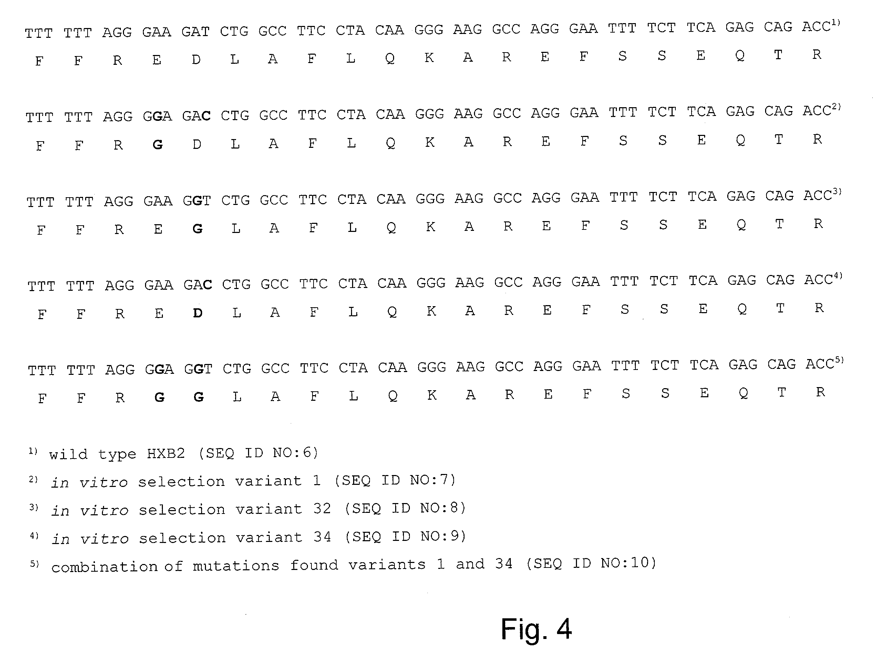

HIV variants according to the invention have in their genome a nucleotide sequence according to any one of the SEQ ID NOS: 2-5 (**Figure 3**) and 7-10 (**Figure 4**).

The HIV variant HXB2-4649-p6 was deposited on 7 June 2004 with the American Type Culture Collection (ATCC) and having the deposit accession number ....

According to the invention HIV variants may in addition one or more of the other mutations, such as those mutations that are present in HIV variant HXB2-4649-p6 or in variants obtained after treatment of wild type or other HIV strains with increasing concentrations of RO033-4649. Examples of additional mutations are listed in Table 1.

The invention also relates to a diagnostic assay for determining the presence in a sample of an HIV-1 variant that is resistant against protease inhibitors, which method comprises:
a) amplification of at least the part of an HIV viral nucleic acid in the sample that contains nucleotide positions 2095, 2098 and 2099;
b) sequence analysis of the amplified viral nucleic acid or part thereof; and
c) comparison of the sequence of the amplified nucleic acid or part thereof with a reference wild type sequence to establish whether one or more of the nucleotides in positions 2095, 2098 and 2099 are mutated.

The mutations are preferably A2095G, A2098G or T2099C.

The skilled person is well capable of selecting a suitable amplification and sequencing technique for use in the invention without undue burden. Such techniques have meanwhile become standard practice. Suitable examples are RT-PCR, NASBA, Real-Time PCR.

The same applies to design of the primers used in the amplification. The entire genome of HIV has been sequenced and the sequence information is readily available. The skilled person will be very well able to design a primer pair that will lead to amplification of that part of the HIV genome that encompasses nucleotides 2095, 2098 and 2099. An example of a primer pairs that can be used for amplification is 5'-clea-2 (5'-AAA TGA TGA CAG CAT GTC AGG G-3'; 1823-1844) and 3'-prot-2 (5'-AAT GCT TTT ATT TTT TCT TCT GTC AAT GGC-3'; 2647-2618).

Other diagnostic assays according to the invention can be phenotypic assays in which sample DNA of a patient's HIV is tested against RO033-4649 and optionally other drugs to assess whether the virus is susceptible or resistant to these drugs.

Diagnostic assays for HIV like the one claimed are traditionally performed on serum and plasma, but other bodily fluids such as whole blood can also be used provided that the HIV population is present therein.

The invention further relates to a method of evaluating the effectiveness of an antiviral therapy of an HIV-infected individual, comprising:
a) determining whether a sample from an HIV-infected individual comprises at least one of the following nucleic acids:
   i) a nucleic acid in which one or more of the nucleotides in positions 2095, 2098 and 2099 are mutated;
   ii) a nucleic acid encoding a HIV-1 p1 of which at least amino acids 436 and/or 437 are mutated;
   iii) the part of nucleic acid of ii) that encodes a p1 fragment of which at least amino acids 436 and/or 437 are mutated;
b) using the presence of said at least one nucleic acid to evaluate the effectiveness of said antiviral therapy.

In case the HIV population of the individual tested comprises viruses having the above mutations it is possible that therapy with the RO033-4649 PI or other PIs will not be effective. In that case alternative treatment can be sought. Here again the at least one mutation of said nucleic acid is preferably selected from A2095G, A2098G or T2099C resulting in mutations selected from K436E in p1, I437T or I437V in p1, or E4G and D5G in TFP (TransFrameProtein).

The information that is obtained from the HIV variants of the invention can be used in the development or identification of novel drugs. Thus, the invention further relates to a method of identifying a drug effective against strains of HIV that are resistant against RO033-4649 and other PIs, comprising:
a) providing an HIV-1 variant comprising at least one mutation in amino acid positions 436 and 437 of p1;
b) contacting the variant with one or more drugs to be tested;
c) determining a phenotypic response of said variant to said drug or drugs; and
d) using said phenotypic response to determine the effectiveness of said drug or drugs.
   The phenotypic response is preferably inhibition of viral growth. Such phenotypic response can for example be determined by using the MTT assay or one of the other assays that are well known to the person skilled in the art. The MTT assay is known from Boucher, C. A. et al. (1996) Antimicrob. Agents Chemother. 40:2404-2409.
   Once identified the drug can be produced and used for the treatment of HIV disease. This method for producing a drug for use in the treatment of HIV disease is also part of this invention and comprises identifying an effective drug by means of the method as claimed, which method further comprises the step of producing the thus identified drug.
   According to another aspect thereof, the invention relates to the drugs identified using the method of the invention and produced according to the invention.
   Once it is known how a resistant HIV strain can be identified, i.e. when his signature mutations are known, this information can be used to design a therapy for treatment. The invention relates to a method therefor, comprising
i) determining whether a sample from an HIV-infected individual comprises at least one nucleic acid chosen from:
   i) a nucleic acid in which one or more of the nucleotides in positions 2095, 2098 and 2099 are mutated;
   ii) a nucleic acid encoding a HIV-1 p1 of which at least amino acids 436 and/or 437 are mutated;
   iii) the part of nucleic acid of ii) that encodes a p1 fragment of which at least amino acids 436 and/or 437 are mutated;
   b) using the presence of said at least one nucleic acid to design the therapy for said patient by selecting drugs that are effective against the resistant variant. The at least one mutation of said nucleic acid is selected from A2095G, A2098G or T2099C. At the amino acid level the at least one mutation of said p1 or p1 fragment is selected from K436E in p1, I437T or I437V in p1.

A method that is somewhat similar is the method for determining the sensitivity of an HIV population circulating in an individual for the drug RO033-4649, comprising
a) amplifying a nucleotide sequence of HIV genetic material from a sample from an individual, which sequence comprises at least the nucleotides at position 2095, 2098 and 2099;
b) identifying mutations that are present in this sequence as compared to the wild type sequence;
d) identifying if the mutation identified matches one or more of the mutations A2095G, A2098G or T2099C. If the mutations match it may well be the case that treatment with RO033-4649 will be less effective and alternatives may be sought.

RO033-4649 as used in this invention is a compound of the general formula:

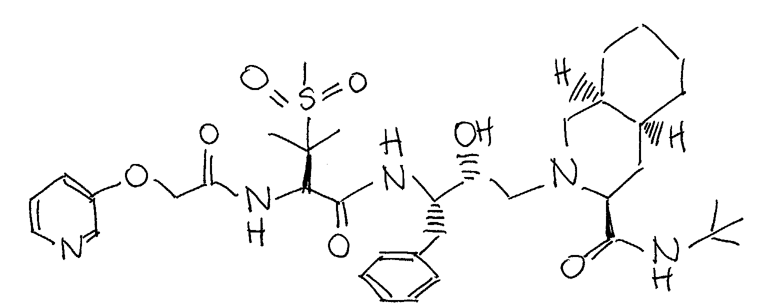

It was disclosed on a poster at the XII International HIV Drug Resistance Workshop held in Cabo Del Sol, Los Cabos, Mexico on June 101-4, 2004. The title of the poster was "*In vitro* cross resistance profile of RO033-4649 against a panel of multiply substituted protease inhibitor resistant viruses: role of common protease resistance mutations" by G. Heilek-Snyder.

The present invention will be further illustrated in the Examples that follow and that are in no way intended to limit the scope of the invention.

In the Examples reference is made to the following Figures.
**Figure 1:** Results of *in vitro* selection with Ritonavir and RO033-4649.
**Figure 2:** Genotypic analysis of mutations in resistant clones.
**Figure 3:** overview of mutations as found in one of the HIV variants of the invention at the nucleic acid and amino acid level located at the p7/p1 cleavage site.
**Figure 4:** overview of mutations as found in one of the HIV variants of the invention at the nucleic acid and amino acid level at the p7/TFP and the TFP/pol6 cleavage site.

### EXAMPLES

### EXAMPLE 1

### Production of resistant viral population

### 1. Cells

SupT1 and MT-2 cells were maintained in RPMI 1640 medium with L-glutamine(BioWhittaker, Verviers, Belgium) supplemented with 10% fetal bovine serum (FBS; Gibco, Breda, The Netherlands) and 10 mg gentamycine (Gibco) per ml and were passaged twice a week. 293 T cells were maintained in Dulbecco's modified Eagle's medium (BioWhittaker, Verviers, Belgium) supplemented with 10% FBS and 10 mg gentamycine and passaged twice a week.

### 2. In vitro selection experiments

Multiple in vitro selection experiments were performed using HIV-1 HXB2 in the presence of increasing concentrations of RO033-4649 or Ritonavir (control experiment). The selection was initiated by infection of SupT1 cells (MOI = 0.01) in the absence of the drug. After one hour incubation at 37°C, culture medium supplemented with the drug was added to a final volume of 10 ml. During the experiment the culture was monitored daily for the appearance of syncytia. When full-blown syncytia were observed, cell free virus was used to initiate the next cell culture passage.

The RO033-4649 drug concentration was raised during the subsequent passages: 20nM in passage 1, 40nM in passage 2, 70nM in passage 3, 80nM in passage 4, 100nM in passage 5 and finally 120nM in passage 6. For Ritonavir the drug concentration was raised accordingly: 60nM in passage 1, 120nM in passage 2, 240nM in passage 3, 480nM in passage 4 and finally 960nM in passage 5 and 6.

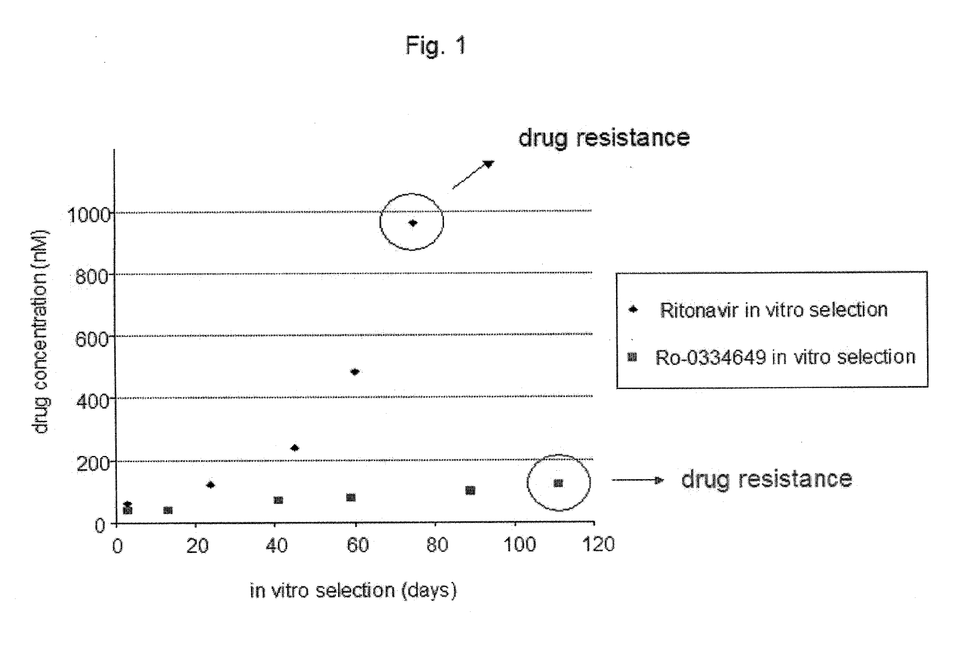

**Figure 1** shows the result of the in vitro selection experiments. In three experiments viral populations were found that were able to replicate in the presence of 120nM RO033-4649.

HIV-1 RNA from the virus supernatant of passage 6 was used for genotypic analysis of the protease, the cleavage sites and gag (Example 2).

### EXAMPLE 2

### Genetic analysis of induced mutations

### 1. Viral RNA analysis

### 1.1 Amplication of the viral protease and cleavage sites

Viral RNA was isolated using the Nuclisens™ Isolation Kit (Organon Teknika, Boxtel, The Netherlands). Briefly, 100 ml sample was mixed with 900 ml lysis buffer and 50 ml silica and incubated for 10 minutes at room temperature to allow binding of the nucleic acid to the silica particles. Unbound material was removed by several washing steps, after which the RNA was eluted in 100 ml of 40 ng/ml poly (A) RNA.

The extracted HIV-1 RNA was then used to reverse transcribe and amplify protease and cleavage sites (PR and CS), essentially as described before (Nijhuis, M. et al. (1999) AIDS 13:2349-2359).

RT-PCR was conducted with primers 5'-clea-1 (5'-GAT GAC AGA AAC CTT GTT GGT CC -3'; 1737-1759) and 3'-prot-1 (5'-GCA AAT ACT GGA GTA TTG TAT GGA TTT TCA GG-3'; 2733-2702). cDNA synthesis was carried out for 30 min at 42°C, followed by RT inactivation for 5 min at 95°C.

Subsequently, a total of 35 cycles were performed, consisting of a denaturation step for 30 sec at 95°C, an annealing step for 30 sec at 50°C and an extension step for 2 min at 72°C.

After this procedure the amount of amplified product was further increased in a second (nested) amplification using primers 5'-p2 (5'-AAG CAA TGA GCC AGG TAA CCA ATT C-3'; 1883-1907) and 3'-prot-2 (5'-AAT GCT TTT ATT TTT TCT TCT GTC AAT GGC-3'; 2647-2618). Primer 5'-p2 contained two nucleotide changes to create a *Bst* EII site in the PCR-fragment. DNA was denatured for two minutes at 94°C, followed by 30 cycles of denaturation at 94°C for 30 sec, annealing at 55°C and extension at 72°C for 2 min.

In order to amplify also the capsid-p2 CS for sequencing analysis the same nested-PCR was performed using primer 5'-clea-2 (5'-AAA TGA TGA CAG CAT GTC AGG G-3'; 1823-1844) instead of 5'-p2.

To amplify the other protease cleavage sites three different RT-PCRs were conducted with either primers 5'-MA-1 (5'-TAG TAT GGG CAA GCA GGG AG-3'; 890-909) and 3'-MA-3 (5'-CAT CCA TCC TAT TTG TTC CTG AAG-3'; 1539-1516) to amplify the matrix-capsid CS, or with primers 5'-NEF-1 (5'-GCA GTA GCT GAG GGG ACA GA-3'; 8685-8704) and 3'-NEF-1 (5'-CCC TGG TGT GTA GTT CTG C-3'; 9186-9168) to amplify the CS in NEF, or with primers 5'RT-INT3 (5'-TAT TAT GAC CCA TCA AAA GAC TTA ATA GC-3'; 3501-3529) and 3'RT-INT3 (5'-AAT GTG TAC AAT CTA GTT GCC ATA TTC-3'; 4431-4405) to amplify the p51/p66 and the p66-integrase CS. Synthesis of cDNA was carried out for 30 min at 42°C, followed by RT inactivation for 5 min at 95°C. Subsequently, a total of 15 cycles were performed, consisting of a denaturation step for 30 sec at 95°C, an annealing step for 30 sec at 60°C and an extension step for 1.5 min at 72°C. Thereafter a total of 20 cycles were performed, consisting of a denaturation step for 30 sec at 95°C, an annealing step for 30 sec at 60°C and an extension step for 1.5 min at 72°C, which was elongated for 5 seconds during each cycle.

To amplify the C-terminal part of 5'LTR and the N-terminal part of the matrix gene, RT-PCR was conducted as described previously for PR using primers HXB2 startfor (5'-GCT AAC TAG GGA ACC CAC TGC TT-3'; 497-519) and HXB2 gagrev (5'TGT GTT TAG CAT GGT GTT TAA ATC TTG-3'; 1359-1333).

To amplify the C-terminal part of the capsid gene, RT-PCR was conducted as described previously for PR using primers cleams-1 (5'-GAA GGA GCC ACC CCA CAA GAT-3'; 1318-1348) and cleamsrev-1 (5'-CCT TAT GGC CGG GTC CTC CTA CT-3'; 1867-1845).

To amplify gp41, RT-PCR was conducted as described previously for PR using primers ENV-5 (5'-CTG ACG GTA CAG GCC AGA CA-3'; 7833-7852) and NEF-3 (5'-TTT GAC CAC TTG CCA CCC AT-3'; 8816-8797).

All PCR-amplified products were purified using the QIAquick PCR purification kit (Qiagen, Leusden, The Netherlands). PCR products were sequenced using the BigDye® Terminator v1.1 Cycle Sequencing Kit (Applied Biosystems, Foster City, CA, USA).

For sequencing of PR and CS, primers 5-clea-4 (5'-ATA ATG ATG CAG AGA GG-3'; 1915-1931), 5'clea-2, 3'CS-2 (5'-TCT CTT CTG GTG GGG CTG TTG GCT C-3'; 2173-2149), PR5 (5'-AGC CAA CAG CCC CAC CAG-3'; 2150-2163) and PR2 (5'-CTT TTG GGC CAT CCA TTC-3'; 2610-2593) were used.

For sequencing of the matrix-capsid CS, primers 5'-MA-2 (5'-TAT AAT ACA GTA GCA ACC CTC TAT TGT G-3'; 1024-1051) and 3'-MA-2 (5'-TCT GCA GCT TCC TCA TTG ATG GT-3'; 1421-1399) were used.

For sequencing of the CS in NEF, primers 5'-NEF-2 (5'-ATG GGT GGC AAG TGG TCA AA-3'; 8797-8817) and 3'-NEF-2 (5'-TCC AGT CCC CCC TTT TCT TT-3'; 9089-9070) were used.

For sequencing of the p51/p66 and p66/integrase CS, primers 5'RT-INT2 (5'-AGC CAC CTG GAT TCC TGA GT-3'; 3770-3789) and 3'RT-INT2 (5'-ATC ACA GCT GGC TAC TAT TT-3'; 4352-4333) were used.

For sequencing the C-terminal part of 5'LTR and the N-terminal part of the matrix gene, primers S667 (5'-CTT AAG CCT CAA ATA AGC TTG AA-3'; 517-539) and 5'LTR-3 (5'-CAG CCT TCT GAT GTT TCT AA-3'; 959-940) were used.

For sequencing the C-terminal part of the capsid gene, primers SK145 (5'-AGT GGG GGG ACA TCA AGC AGC CAT-3'; 1359-1382) and cleamsrev-2 (5'-TTC TTC TAG TGT AGC CGC TGG TCC-3'; 1824-1801) were used.

For sequencing gp41, primers ENV-6 (5'-ACA ATT ATT GTC TGG TAT AG-3'; 7850-7870), ENV-7 (5'-CCT TTC CAA GCC CTG TCT TA-3'; 8783-8764), ENV-8 (5'-TAT CCC TGC CTA ACT CTA TTC A-3'; 8359-8338) and ENV-9 (5'-GTT TAA GAA TAG TTT TTG CTG TAC TTT-3'; 8305-8331) were used.

**Table 1** shows the mutations that were identified in experiment 1. Of these the mutations in nucleotide positions 2095 and 2099 are relevant as it was demonstrated that they confer 2-5 fold resistance against all known HIV protease inhibitors.

**Table 1**

| Nt* | HXB2 Athena | P6 (0.12 µM = 3x IC₅₀) | Gene | Amino Acid position | Cleavage site | Pos. | Amino Acid change |
|---|---|---|---|---|---|---|---|
| 609 | T | W | 5' LTR U5 | - | - | - | - |
| 907 | G | A | Matrix | 40 | | | E->K |
| 1126 | A | R | Matrix | 113 | | | K->K/E |
| 1460 | C | Y | Capsid | 92 | | | A->A/V |
| 2095 | A | G | | | p7/p1 | P4' | K->E |
| | | | | | p7/TFP | P4' | E->G |
| | | | | | TFP/p6 pol | P5 | E->G |
| 2099 | T | C | | | p7/p1 | P5' | I->T |
| 2180 | G | R | p6 gag | 16 | | | R->R/K |
| 8101 | C | Y | gp41 | 105 | | | T->T/M |

| | | | | | | | |
|---|---|---|---|---|---|---|---|
| * Nucleotide positions in regard with HXB2 | | | | | | | |

In another *in vitro* selection experiment (32) the mutation A2098G was found. Mutation T2099C was also found in experiment 34.

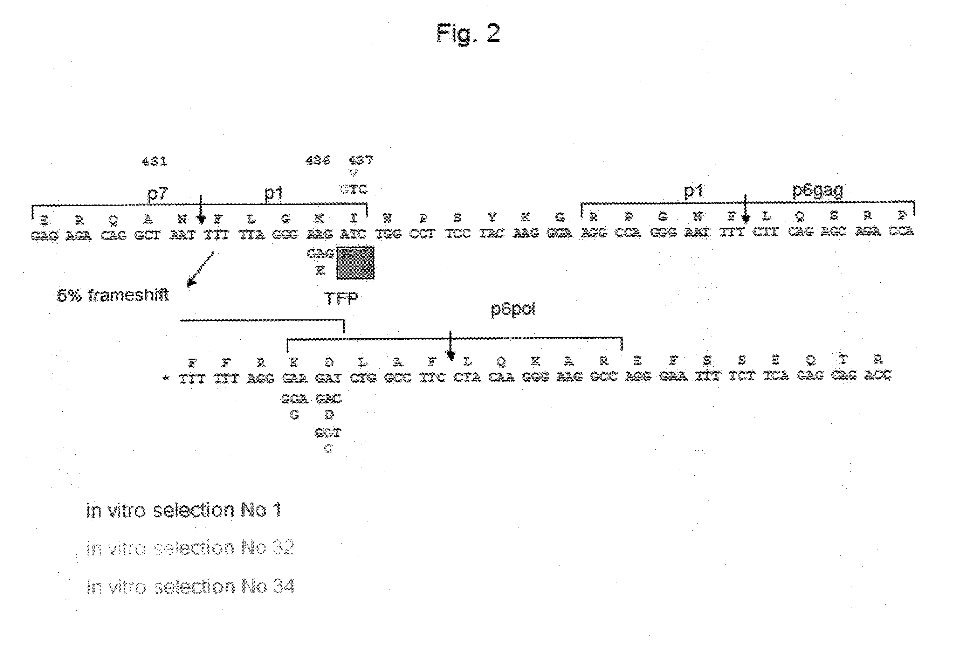

**Figure 2** shows the effect of the nucleotide mutations located at the protease cleavage sites on the amino acid sequence.

### 2. Construction of PR and CS-deleted vector

To construct a novel HXB2 deletion clone lacking PR and the p2/p7, p7/p1 and p1/p6 CS an HXB2 deletion clone lacking PR (pHXBΔPR) was used (Maschera, B. et al. (1995) J. Virol. 69:5431-5436.). To create a unique *Mlu* NI site at the 5' end of the RT gene of pHXBΔPR, the *Mlu* NI site present at the integrase gene had to be deleted. Therefore pHXBΔPR was digested with *Psh* AI and *Nco* I to remove a 1283 bp fragment containing the Mlu NI site. PCR, using Vent_{R}® DNA polymerase (New England BioLabs, Frankfurt am Main, Germany), was performed on pHXBΔPR with primers INT-Psh AI (5'-GCA TGG ACA AGT AGA CTG TAG TC-3'; 4379-4401, Invitrogen, Breda, The Netherlands), INT-Nco I (5'-TGT TGC CCT AAG CCA TGG AG-3'; 5692-5673) and INT-Mlu NI (5'-TGT ATT GTT TTT ACC GGC CAT CTT-3'; 4570-4547). Primer INT-Mlu NI contained one silent nucleotide change to delete the *Mlu* NI site in the PCR-fragment. This PCR fragment was then digested with *Psh* AI and *Nco* I and ligated with the digested pHXBΔPR. Sequence analysis was performed to confirm correct plasmids.

Subsequently the CS had to be removed from pHXBΔPR. Therefore pHXBΔPR was digested with *Spe* I and *Bst* EII to remove the p2/p7, p7/p1 and p1/p6 CS. However, also a fragment of 400 bp before the p2/p7 CS was removed. To restore this fragment, PCR was performed on pHXBΔPR using primers p24 WT (5'-GCA GGA ACT ACT AGT ACC CTT CAG GAA CAA ATA G-3'; 1498-1531) and p2 *Bst* EII Mut (5'-TAG CTG AAT TGG TTA CCT GGC TCA TTG-3'; 1912-1886). Primer p2 Bst EII Mut contained two silent nucleotide changes with respect to pHXBDPR, thereby introducing a *Bst* EII site in the PCR fragment. The PCR fragment was then digested with *Spe* I and *Bst* EII and ligated with the digested pHXBΔPR, resulting in the wild type vector. To confirm that the vector was correct, sequence analysis was performed.

### 3. Generation of recombinant virus clone

To generate a clone containing the PR and the mutated CS, the PCR product was recombined with the HXB2 vector. Therefore the PCR-product and vector were digested with *Bst* EII and *Mlu* NI. PCR-product and vector were ligated using the Rapid DNA Ligation Kit (Roche Diagnostics GmbH, Mannheim, Germany). During ligation, 1 ml of 1:5 diluted Asp I (Roche) was added to digest incorrect plasmids, which contain an unique Asp I restriction site, to prevent that those incorrect plasmids would be transformed.

Subsequently, the ligation product was transformed into *E.coli* JM109 High Efficiency Competent Cells (Promega, Madison, USA) by means of a heat-shock at 42°C. After transformation 450 ml Luria-Bertani (LB) medium was added to the competent cells and the cells were incubated at 37°C for 1 hour. LB-agar plates, containing 40 mg/ml ampicillin (AMP), were prepared and 100 ml medium and cells was spread on a LB-agar plate. After overnight incubation at 37°C, plasmid was isolated from a single colony using the QIAGEN Plasmid Mini Kit (Qiagen).

Subsequently, the isolated plasmid was used to transfect 293T cells. Therefore, 5-6×10⁶ 293T cells were seeded the day prior to transfection to achieve 90-95% confluence on the day of transfection. For transfection of 10 mg of plasmid DNA, Lipofectamine 2000 (Invitrogen) was used according to the manufacturer's protocol. Two days after transfection recombinant virus was harvested.

### 4. Drug susceptibility analysis

The infectious virus titre (TCID₅₀) of the complete virus variant and the PCR clone was determined using end-point dilutions in MT2 cells (Reed, L. J. and H. Muench (1983) Am J Hyg 493-497). Drug susceptibility of the viruses was determined in duplicate using an MTT assay (Boucher, C. A. et al. (1996) Antimicrob. Agents Chemother. 40:2404-2409).

Table 2 shows the results.

**Table 2**

| **drug** | **resistance compared to HXB2** |
|---|---|
| *Protease inhibitors* | |
| RO033-4649 (invention) | 2-3-fold |
| Ritonavir | 2-5-fold |
| Indinavir | 2-5-fold |
| Nelfinavir | 2-5-fold |
| Saquinavir | 2-5-fold |
| Amprenavir | 2-5-fold |
| Atazanavir | 2-5-fold |
| Tipranavir | 2-5-fold |
| Lopinavir | 2-5-fold |

## Claims

1. Human Immunodeficiency Virus-1 (HIV-1) variants in isolated form showing resistance against protease inhibitors and having at least one or more of the following mutations at the DNA level as compared to HIV-1 HXB2: A2095G, A2098G and T2099C.

2. HIV variants as claimed in claim 1, having at least one or more of the following mutations at the amino acid level as compared to HIV-1 HXB2: K436E in p1, I437T or I437V in p1.

3. HIV variants as claimed in claim 1, having at least one or more of the following mutations at the amino acid level as compared to HIV-1 HXB2: E4G and D5G in the TransFrameProtein.

4. HIV variant as claimed in claim 1 or 2, having in its genome a nucleotide sequence according to any one of the SEQ ID NOS: 2-5 (**Figure 3**) and 7-10 (**Figure 4**).

5. HIV variant HXB2-4649-p6 as deposited on 7 June 2004 with the American Type Culture Collection (ATCC) and having the deposit accession number ....

6. HIV variant as claimed in any one of the claims 1-4 having in addition one or more of the other mutations that are present in HIV variant HXB2-4649-p6.

7. Diagnostic assay for determining the presence in a sample of an HIV-1 variant that is resistant against protease inhibitors, which method comprises:
a) amplification of at least the part of an HIV viral nucleic acid in the sample that contains nucleotide positions 2095, 2098 and 2099;
b) sequence analysis of the amplified viral nucleic acid or part thereof; and
c) comparison of the sequence of the amplified nucleic acid or part thereof with a reference wild type sequence to establish whether one or more of the nucleotides in positions 2095, 2098 and 2099 are mutated.

8. Diagnostic assay as claimed in claim 7, wherein the mutations are A2095G, A2098G or T2099C.

9. Diagnostic assay as claimed in claim 7 or 8, wherein the amplification takes place by means of RT-PCR, NASBA or Real Time PCR.

10. Diagnostic assay as claimed in any one of the claims 6-9, wherein the primer pair that is used for amplification is 5'-clea-2 (5'-AAA TGA TGA CAG CAT GTC AGG G-3'; 1823-1844) and 3'-prot-2 (5'-AAT GCT TTT ATT TTT TCT TCT GTC AAT GGC-3'; 2647-2618).

11. Diagnostic assay as claimed in any one of the claims 6-10, wherein the sample is selected from serum, plasma, whole blood.

12. Method of evaluating the effectiveness of an antiviral therapy of an HIV-infected individual, comprising:
a) determining whether a sample from an HIV-infected individual comprises at least one of the following nucleic acids:
i) a nucleic acid in which one or more of the nucleotides in positions 2095, 2098 and 2099 are mutated;
ii) a nucleic acid encoding HIV-1 p1 of which at least amino acids 436 and/or 437 are mutated;
iii) the part of nucleic acid of ii) that encodes a p1 fragment of which at least amino acids 436 and/or 437 are mutated;
b) using the presence of said at least one nucleic acid to evaluate the effectiveness of said antiviral therapy.

13. The method according to claim 12 wherein said at least one mutation of said nucleic acid is selected from A2095G, A2098G or T2099C.

14. The method according to claim 12 wherein said at least one mutation of said p1 or p1 fragment is selected from K436E in p1, I437T or I437V in p1.

15. Method of identifying a drug effective against RO033-4649 resistant strains of HIV, comprising:
a) providing an HIV-1 variant containing at least one mutation in amino acid positions 436 and 437 of p1;
b) contacting the variant with one or more drugs to be tested;
c) determining a phenotypic response of said variant to said drug or drugs; and
d) using said phenotypic response to determine the effectiveness of said drug or drugs.

16. Method as claimed in claim 15, wherein the phenotypic response is inhibition of viral growth.

17. Method as claimed in claim 16, wherein the phenotypic response is determined by using the MTT assay.

18. Method for producing a drug for use in the treatment of HIV disease, comprising identifying an effective drug by means of the method as claimed in any one of the claim 15-17, which method further comprises the step of producing the thus identified drug.

19. A drug identified using the method of any one of the claim 15-17.

20. A drug produced using the method of claim 18.

21. A method of designing a therapy for treating a patient infected with HIV, comprising:
a) determining whether a sample from an HIV-infected individual comprises at least one nucleic acid chosen from:
i) a nucleic acid in which one or more of the nucleotides in positions 2095, 2098 and 2099 are mutated;
ii) a nucleic acid encoding HIV-1 p1 of which at least amino acids 436 and/or 437 are mutated;
iii) the part of nucleic acid of ii) that encodes p1 fragment of which at least amino acids 436 and/or 437 are mutated;
b) using the presence of said at least one nucleic acid to design the therapy for said patient by selecting drugs that are effective against the resistant variant.

22. The method according to claim 21 wherein said at least one mutation of said nucleic acid is selected from A2095G, A2098G or T2099C.

23. The method according to claim 21 wherein said at least one mutation is selected from K436E in p1, I437T or I437V in p1.

24. A method for determining the sensitivity of an HIV population circulating in an individual for the drug RO033-4649, comprising
a) amplifying a nucleotide sequence of HIV genetic material from a sample from an individual, which sequence comprises at least the nucleotides at position 2095, 2098 and 2099;
b) identifying mutations that are present in this sequence as compared to the wild type sequence;
d) identifying if the mutation identified matches a one or more of the mutations A2095G, A2098G or T2099C.
